# EUROPÄISCHE PATENTSCHRIFT

(11) **EP 0 371 355 B1**
(45) Veröffentlichungstag und Bekanntmachung des Hinweises auf die Patenterteilung: **21.04.1993**
(21) Anmeldenummer: 89121291.2
(22) Anmeldetag: 17.11.1989
(51) Int. Cl.: G01N 33/00

(54) **Verfahren zur Überwachung des Gasstromes in einem Gasanalysator und zur Korrektur von druck- und durchflussbedingten Störeinflüssen auf dessen Messsignal**
Process for supervising the flow of gas in a gas analyser and for correcting the interfering effects of pressure and flow on the measure signal
Procédé pour surveiller de débit de gaz dans un analyseur de gaz et pour corriger les influences parasites de la pression et du débit sur le signal de mesure

(30) Priorität: 30.11.1988 DE 3840322
(43) Veröffentlichungstag der Anmeldung: 06.06.1990
(73) Patentinhaber: Hartmann & Braun Aktiengesellschaft, 60484 Frankfurt am Main (DE)
(72) Erfinder: Fabinski, Walter, D-6239 Kriftel (DE); Taubitz, Georg, Dr., D-6370 Oberursel (DE); Franck, Gerhard, Dr., D-6000 Frankfurt 90 (DE); Nevole, Josef, D-6000 Frankfurt 1 (DE)
(74) Vertreter: Meissner, Peter E., Dipl.-Ing.

(56) Entgegenhaltungen:
- EP-A- 0 028 826
- FR-A- 1 548 994
- GB-A- 2 094 483
- US-A- 4 056 967

## Beschreibung

Die Erfindung betrifft ein Verfahren zur Korrektur von Störeinflüssen auf die Strömungsmengenmessung des Gasstromes in einem Gasanalysator.

Bei Gasanalysatoren mit kontinuierlicher Beströmung mit dem Meßgas wird der Gasstrom üblicherweise überwacht mit Einrichtungen zur Messung und Anzeige des Gasvolumen- oder Massenstromes; im folgenden als Strömungsmengenmessung bezeichnet.

Insbesondere für automatisch arbeitende Gasanalysatoren ohne ständige Beobachtung ist es vorteilhaft, die kontinuierliche Strömungsmengenmessung durch eine Überwachung von Grenzwerten zu ergänzen, d.h. Überwachen auf Überschreiten einer Mindestströmungsmenge bzw. Unterschreiten einer maximalen Strömungsmenge, wobei beim Verlassen des erlaubten Strömungsmengenbereichs eine besondere Signalgabe erfolgt, um z.B. Wartungseingriffe auszulösen.

Bestimmte Gasanalysatoren reagieren aufgrund ihres physikalischen Meßprinzips auf Druckveränderungen in ihrer Meßkammer oder -küvette mit einer Veränderung ihres Meßsignals. Für genaue Messungen muß entweder der absolute Meßkammerdruck konstantgehalten werden, oder das Analysatormeßsignal muß eine druckabhängige Korrektur erfahren.

Für einfachere Gasanalysenmeßeinrichtungen werden vorwiegend mech.-pneumat. Meßeinrichtungen eingesetzt, um Gasströmungen zu messen und zu überwachen. Dazu gehören Schwebekörperströmungsmesser mit einem Schwebekörper (z.B. einer Kugel) in einem konisch kalibrierten Glasrohr. Die Schwebehöhe der Kugel zeigt die Strömungsmenge an einer Skala an. Die gewünschte Überwachung und Signalisierung einer abweichenden Strömungsmenge geschieht durch Festlegung eines unteren bzw. oberen Wertes. Diese werden so ermittelt, daß induktive, optische oder magnetische Abgriffsysteme um das Glasrohr gelegt werden, die bei Erreichen der Grenzwerte aufgrund einer Wechselwirkung mit dem Schwebekörper ein Signal auslösen (DE-AS 25 04 275, DE-OS 14 98 896).

Eine weitere einfache Möglichkeit zur Grenzwertüberwachung besteht in der Verwendung eines pneumatisch-elektrischen Druckschalters, der auf Veränderungen im statischen Druck des Gasanalysators reagiert (DE-AS 12 11 423).

Andere pneumatische Meßverfahren nutzen den an einer Meßblende oder -kapillare entstehenden Differenzdruck zur Messung des Durchflusses aus (DE-OS 15 23 019, DE-OS 19 03 353).

Gasströmungen können auch mit kalorimetrischen Systemen ermittelt und angezeigt werden. Hierbei benutzt man elektrisch beheizte Drähte, die durch die Gasströmung gekühlt werden. Die Abkühlung der Drähte ändert deren Widerstand. Diese Widerstandsänderung wird elektrisch weiterverarbeitet (DE-OS 21 04 894).

Bei allen Überwachungsverfahren mit elektrischem Meßsignal findet eine normierte oder physikalische Anzeige des Durchflusses sowie eine Grenzwertüberwachung durch Vergleich mit festlegbaren Sollwerten in der bekannten Technik statt.

Zur Messung des Druckes in der Meßkammer (-küvette) zur Korrektur von druckabhängigen Einflußeffekten auf das Meßsignal sind u.a. piezoresistive Geber geeignet. Deren Meßsignal greift in bekannter Weise in die elektrische Signalverarbeitung des Analysengerätes ein.

Aus dem Dokument FR-A-15 48 994 ist eine Anordnung zur Messung der relativen Feuchte eines Gases bekannt, bei der zur Messung und Überwachung eines Gasstromes, der durch eine Meßkammer befördert wird, ein Strömungswiderstand und zwei Druckaufnehmer vorgesehen sind. Der Strömungswiderstand ist in der Gasleitung hinter der Meßkammer und ein erster Druckaufnehmer ist innerhalb der Meßkammer angeordnet. Der zweite Druckaufnehmer befindet sich in einem Trockner, der der Meßkammer vorgelagert ist.

Die bekannten Anordnungen benutzen stets mindestens zwei Meßwertaufnehmer für die folgenden drei Meßaufgaben
- Strömungsmengenmessung und -anzeige,
- Grenzwertüberwachung der Strömungsmenge und
- Druckmessung in der Meßkammer bzw -küvette zur Korrektur von druckabhängigen Analysatormeßfehlern,
was zu einem relativ hohen technischen Meßaufwand und einer entsprechend großen Störanfälligkeit führt.

Es stellt sich die Aufgabe, den Aufwand mit Hilfe eines Korrekturverfahrens zu verringern, welches die vorgenannten drei Meßaufgaben unter Verwendung eines einzigen Meßwertaufnehmers erfüllt. Diese Aufgabe wird durch die im Kennzeichen des Anspruches gegebene Lehre gelöst.

Nach bekannten Gesetzen der Strömungslehre erzeugt der Gasstrom durch die Meßkammer und die Gasleitungen des Gasanalysators einen in funktionalem Zusammenhang mit der Größe des Volumenstroms stehenden Druckanstieg vor dem Strömungswiderstand.

Mißt man den Druck vor diesem Strömungswiderstand als Absolutdruck, der gleichzeitig auch die geodätische Höhe und die barometrischen Druckschwankungen erfaßt, so hat man die Möglichkeit, mit einer einzigen Meßgröße bei entsprechender Signalauswertung alle drei Funktionen Strömungsmengenmessung, Grenzwertüberwachung und Korrektur des druckabhängigen Analysator-Meßfehlers auf einfache Weise durchzuführen. Die Signalauswertung und Korrekturrechnung wird vorteilhaft von einem Mikroprozessor vorgenommen, wie er in modernen Analysengeräten meist ohnehin vorhanden ist.

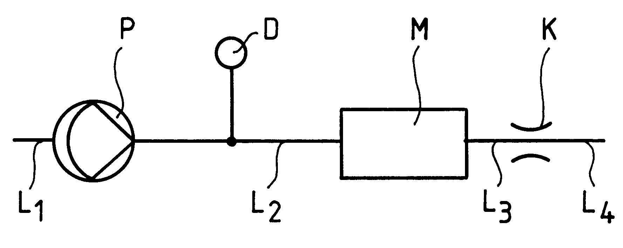

Ein Ausführungsbeispiel der Erfindung ist in der Zeichnung dargestellt und wird im folgenden näher beschrieben.

Das Meßgas durchströmt die Gasleitung L₁ und wird durch eine Pumpe P über die Leitung L₂ der Küvette oder der Meßkammer M eines Analysators zugeführt. Hinter der Meßkammer M ist als Strömungswiderstand K eine Kapillare oder Blende in die Gasleitung L₃ geschaltet, die in Abhängigkeit vom Gasdurchfluß einen Druckanstieg in der Meßkammer erzeugt. Vor dem Strömungswiderstand K ist ein Druckaufnehmer D als Absolutdruckmesser angeordnet, der den Druck des Meßgases in der Gasleitung mißt. Der Druckaufnehmer D kann prinzipiell sowohl in der Meßkammer M selbst, davor oder dahinter eingesetzt werden, wenn die dazwischenliegenden Strömungswiderstände in den Gasleitungen L₂ und L₃ vernachlässigbar sind. Das Signal des Druckaufnehmers wird zur rechnerischen Korrektur des Druckeinflußeffektes auf das Analysatormeßsignal herangezogen.

Da der Druckanstieg als Funktion des Durchflusses nach den Gesetzen der Strömungsmechanik näherungsweise quadratisch erfolgt, reagiert die Absolutdruckmessung recht empfindlich auf Durchflußveränderungen. Als Konstantanteil ist dem Signal des Druckaufnehmers der Einfluß der geodätischen Höhe des Meßortes (wichtig z.B. bei mobilen Messungen oder bei Absolutdruck-Kalibrierstandards) überlagert. Zusätzlich überlagert ist ggf. der in der Abgasleitung L₄ herrschende Druck und als langsam veränderlicher Anteil der Einfluß der barometrischen Druckschwankungen. Die quasistatischen Druckveränderungen bewirken bezüglich der Strömungsmengenmessung mittels Absolutdruckmessung eine Verfälschung des Meßsignals und damit auch der vorgegebenen Strömungsgrenzwerte. Um solche Einflüsse zu eliminieren, wird gemäß dem Anspruch der statische Meßkammerdruck in vorgebbaren Zeitabständen gemessen und zur Korrektur der Strömungsmengenmessung benutzt. Das geschieht durch kurzzeitiges Unterbrechen des Meßgas-Durchflusses, z.B. durch Abschalten der Meßgaspumpe.

Im vorliegenden Fall wurde eine Anordnung gewählt, die bei einer mittl. Strömung von 50 l/h einen oberen Grenzwert von 60 l/h und einen unteren von 40 l/h liefert. Der durch die Strömung bedingte dynamische Druckanteil beträgt dabei ca. 70 mbar bei einer zugrundegelegten Kapillare von 0,7 mm Durchmesser und einer Länge von 15 mm.

## Patentansprüche

1. Verfahren zur Korrektur von Störeinflüssen auf die Strömungsmengenmessung des Gasstromes in einem Gasanalysator infolge von zeitlichen Veränderungen im Druck in der Ausgangsleitung (L₄) einer Gasleitung (L₁ - L₄), unter Verwendung einer Anordnung
- mit einer Pumpe (P) zur Förderung des Gasstromes durch eine Meßkammer (M) des Gasanalysators,
- mit einem Strömungswiderstand (K) in der Gasleitung (L₃) hinter der Meßkammer (M) und
- mit einem Druckaufnehmer (D) in der Gasleitung (L₂) in Strömungsrichtung vor dem Strömungswiderstand (K),
dadurch gekennzeichnet,
- daß in vorgebbaren Zeitabständen der Durchfluß des Gasstromes durch die Meßkammer (M) manuell oder selbstätig unterbrochen wird,
- daß in diesen Unterbrechungszeiten der Druck an der Anschlußstelle des Druckaufnehmers (D), der dann dem statischen Außendruck entspricht, mit Hilfe des Druckaufnehmers (D) gemessen wird und
- daß die so gemessene Größe des statischen Druckes - bestehend aus einer geodätischen und einer barometrischen Komponente - als Referenzdruck zur rechnerischen Korrektur der verfälschenden Einflüsse der quasistatischen Druckveränderungen auf die Messung der Strömungsmenge und die Überwachung der vorgegebenen Strömungsmengen-Grenzwerte herangezogen wird.

## Claims

1. Method for correcting interference with the flow rate measurement of the gas flow in a gas analyser due to variations over time of the pressure in the outlet pipe (L₄) of a gas pipe (L₁ - L₄), using an arrangement
- with a pump (P) for feeding the gas flow through a measurement chamber (M) of the gas analyser,
- with a flow resistance (K) in the gas pipe (L₃) downstream of the measurement chamber (M) and
- with a pressure sensor (D) in the gas pipe (L₂) in the flow direction upstream of the flow resistance (K),
characterised in that
- at pre-settable intervals of time the flow of the gas flow through the measurement chamber (M) is interrupted manually or automatically,
- at said interruption times the pressure at the connection point of the pressure sensor (D), which then corresponds to the static external pressure, is measured by means of the pressure sensor (D) and
- the value thus measured for the static pressure - consisting of a geodetic and a barometric component - is used as a reference pressure for the numerical correction of the distorting effects of the quasi-static pressure changes on the measurement of the flow rate and the monitoring of the pre-set flow rate limit values.

## Revendications

1. Procédé de correction des influences parasites exercées sur les mesures du débit de gaz dans un analyseur de gaz à la suite des variations de la pression dans la section de sortie L₄ de conduite de gaz L₁ à L₄, comportant :
- une pompe (P) faisant traverser au gaz une chambre de mesure (M) de l'analyseur de gaz,
- un frein à l'écoulement (K) monté dans la conduite L₃ faisant suite à la chambre (M),
- un capteur de pression (D) monté dans la conduite de gaz (L₂), en amont du frein à l'écoulement (K)
caractérisé en ce que
- le débit du gaz à travers la chambre de mesure (M) est interrompu, à des intervalle de temps donnés, manuellement ou automatiquement,
- pendant ces interruptions, le capteur de pression (D) mesure la pression à son raccordement à la conduite de circulation du gaz, cette pression correspondant alors à la pression statique externe,
- la grandeur ainsi mesurée de la pression statique, correspondant à l'altitude du lieu de mesure et à la situation barométrique du moment, est prise comme valeur de référence pour calculer la correction des influences parasites exercées par les variations quasistatiques de la pression sur la mesure du débit de gaz et pour assurer la surveillance des valeurs limites imposées au débit.
